# EUROPÄISCHE PATENTSCHRIFT

(11) **EP 2 054 111 B1**
(45) Veröffentlichungstag und Bekanntmachung des Hinweises auf die Patenterteilung: **26.06.2019**
(21) Anmeldenummer: 07785092.3
(22) Anmeldetag: 14.08.2007
(51) Int. Cl.: A61M 5/24, A61M 5/315

(54) **DOSIERMECHANIKSPERRUNG**
BLOCKING ELEMENT FOR A DOSING MECHANISM
BLOCAGE MECANIQUE D'UN DOSEUR

(30) Priorität: 14.08.2006 DE 102006038123; 14.08.2006 DE 202006019890 U; 06.12.2006 DE 102006057578; 22.12.2006 DE 202006019370 U; 09.01.2007 DE 102007001432
(43) Veröffentlichungstag der Anmeldung: 06.05.2009
(73) Patentinhaber: TecPharma Licensing AG, 3401 Burgdorf (CH)
(72) Erfinder: HIRSCHEL, Juerg, 5000 Aarau (CH); MOSER, Ulrich, 3412 Heimiswil (CH); SCHRUL, Christian, 3400 Burgdorf (CH); TSCHIRREN, Markus, 3422 Kirchberg (CH)
(86) Internationale Anmeldenummer: PCT/CH2007/000397
(87) Internationale Veröffentlichungsnummer: WO 2008/019518

(56) Entgegenhaltungen:
- EP-A- 0 554 995
- WO-A-00/62839
- WO-A-02/092153
- DE-C1- 19 821 934

## Beschreibung

Die Erfindung betrifft eine Dosiermechanik einer Injektionsvorrichtung, insbesondere zur Verwendung mit einer Zweikammerampulle, in welcher zwei voneinander getrennte Substanzen enthalten sind, die vor der Verabreichung durch die Injektionsvorrichtung möglichst vollständig abgemischt werden sollen.

Wird eine Zweikammerampulle unvollständig oder nur zum Teil in eine Injektionsvorrichtung eingeschraubt, so besteht die Möglichkeit, dass die in der Zweikammerampulle enthaltenen Substanzen nicht oder nur unvollständig abgemischt werden, wobei bei einem nachfolgenden Injektionsvorgang aus einer nur unvollständig abgemischten Zweikammerampulle nicht die gewünschte Substanz abgegeben wird und beispielsweise auch die nicht abgemischten Ausgangsmaterialien der Zweikammerampulle abgegeben werden.

WO 00/62839 beschreibt einen Injektor mit einem Sperrmechanismus zum Sperren eines Dosierelements, wobei der Sperrmechanismus eine Rippe an einem Knopf und einen Spalt in einer Hülse umfasst. Dieser Sperrmechanismus dient dazu, dass zuerst eine Zweikammerkarpule vollständig abgemischt wird, bevor eine Dosis eingestellt werden kann.

Es ist eine Aufgabe der vorliegenden Erfindung, eine alternative Dosiermechanik für eine Injektionsvorrichtung vorzuschlagen, mit welcher der Einsatz von Injektionsvorrichtungen insbesondere in Verbindung mit Zweikammerampullen sicherer gemachtwird.

Diese Aufgabe wird durch die Gegenstände der unabhängigen Ansprüche gelöst. Vorteilhafte Ausführungsformen ergeben sich aus den abhängigen Ansprüchen.

Eine erfindungsgemäße Dosiermechanik dient zur Sperrung eines Einstell-, Aufzieh- oder Dosierelements einer zum Beispiel als Einweginjektor ausgebildeten Injektionsvorrichtung oder eines Pens und weist mindestens ein bevorzugt bewegbares und insbesondere flexibel ausgebildetes Halteelement auf, welches mit dem Dosierelement der Injektionsvorrichtung so zusammenwirken kann, dass eine Aufzug-, Dosier- oder Einstellbewegung oder ein Einstellvorgang, wie zum Beispiel eine Drehung oder ein Verschieben und zum Beispiel Herausziehen des Dosierelements, in einer Ausgangsposition des Sperrelements zum Beispiel durch eine Verrastung mit dem Sperrelement verhindert werden kann und erst nach einer Verschiebung oder Bewegung des mindestens einen Halteelements, welche zum Beispiel durch ein Verschieben des Sperrelements durch das oder nach dem Einschieben einer Ampulle ausgelöst oder bewirkt wird, ermöglicht wird. Somit kann durch die erfindungsgemäße Dosiermechanik verhindert werden, dass ein Einstell- oder Dosierelement betätigt werden kann, um zum Beispiel eine Dosis einzustellen oder die Injektionsvorrichtung aufzuziehen, bevor eine Ampulle, insbesondere eine Zweikammerampulle, welche in die Injektionsvorrichtung eingeschoben wird, zum Beispiel in Anlage an das Sperrelement kommt oder das Sperrelement einen vorgegebenen Weg von zum Beispiel 2 mm verschoben hat, um eine Freigabe der Dosiermechanik zum Beispiel durch ein Herausbewegen eines in die Dosiermechanik eingreifenden Halteelementes zu bewegen.

Vorzugsweise ist das Sperrelement als Ring ausgebildet und weist vorteilhaft eine Anschlagfläche für eine Ampulle oder eine Ampullenhülse auf, so dass eine in eine Injektionsvorrichtung eingesetzte oder vollständig oder fast vollständig eingedrehte Ampulle in Anlage mit dem Sperrelement kommen kann und dieses zum Beispiel auf dem letzten Teilstück der Einschub- oder Eindrehbewegung relativ zur Injektionsvorrichtung oder relativ zur Einstellmechanik mitnehmen kann. Das mindestens eine Halteelement ist vorzugsweise ein radial nach innen oder radial nach außen vorgespanntes Halteelement, welches beispielsweise in eine Ausnehmung oder Nut eines Dosierelements oder einer Dosiervorrichtung eingreift und zum Beispiel bei einer drehsicheren Lagerung des Sperrelements in der Injektionsvorrichtung eine Drehbewegung oder ein Herausziehen des Dosierelements verhindern kann. Vorteilhaft sind zwei oder mehr Halteelemente vorgesehen, wie zum Beispiel zwei an einem ringförmigen Sperrelement einander gegenüberliegende Halteelemente, welche in einer Ausgangslage bei noch nicht vollständig eingesetzter Ampulle beispielsweise radial nach innen vorgespannt sein können und zum Beispiel in die Dosiermechanik oder ein Dosierelement eingreifen und erst nach Einbringen einer Ampulle radial nach außen geschoben werden, um ein Dosierelement oder eine Dosiermechanik freizugeben.

Die Erfindung bezieht sich auf eine Dosiermechanik für eine Injektionsvorrichtung mit einem wie oben beschriebenen Sperrelement und mindestens einem Dosierelement, wie zum Beispiel einem Drehknopf oder einer Drehhülse. Vorzugsweise weist das Dosierelement mindestens ein Halte- oder Eingriffselement oder eine Ausnehmung, wie zum Beispiel eine Nut auf, mit welchem das mindestens eine Halteelement des Sperrelements zusammenwirken, also zum Beispiel in dieses eingreifen kann. Dabei ist das Sperrelement vorzugsweise verschiebbar und bevorzugt relativ zum Dosierelement axial verschiebbar gelagert, also beispielsweise auf dieses zu oder in dieses hinein oder aus diesem heraus verschiebbar, wobei das mindestens eine Halteelement des Sperrelementes so vorgesehen sein kann, dass bei oder nach einer Verschiebung des Sperrelementes relativ zum Dosierelement das oder die Halteelemente zum Beispiel durch eine nicht zusammen mit dem Sperrelement verschobene Rampe so bewegt oder verschoben werden, dass keine Kopplung mehr zwischen dem Sperrelement der Dosiermechanik oder dem Dosierelement besteht, so dass das Dosierelement oder die Dosiermechanik betätigt und zum Beispiel zum Einstellen einer Dosis oder zum Aufziehen der Injektionsvorrichtung gedreht oder beispielsweise aus der Injektionsvorrichtung herausgezogen werden können.

Allgemein soll unter einem Halteelement im Sinne dieser Erfindung jedes Element oder auch eine Ausnehmung oder Durchbohrung verstanden werden, welche es ermöglichen, dass eine Kopplung, wie beispielsweise eine Verdrehsicherung, mit einem anderen Element hergestellt werden kann. Beispielsweise kann ein bewegbares oder flexibles radial nach innen oder außen vorgespanntes Halteelement an dem Sperrelement der Dosiermechanik vorgesehen sein, welche mit einem anderen Halteelement oder einer Ausnehmung oder zum Beispiel Vertiefung oder Nut an dem jeweiligen Gegenelement, also beispielsweise dem Dosierelement oder der Dosiermechanik bzw. dem Sperrelement, zusammenwirken, um so eine lösbare Kopplung zwischen dem Sperrelement einerseits und dem Dosierelement oder der Dosiermechanik andererseits herzustellen. Diese Kopplung soll vorzugsweise dann gelöst werden, wenn eine Ampulle bis zu einer vorgegebenen Länge in die Injektionsvorrichtung eingebracht wurde, wobei die Lösung der Kopplung zum Beispiel durch ein durch das Einbringen der Ampulle bedingtes und zum Beispiel durch ein Führungsprofil geführtes Verschieben mindestens eines Halteelementes bewirkt werden kann.

Gemäß einem weiteren Aspekt bezieht sich die Erfindung auf eine Injektionsvorrichtung mit einer wie oben beschriebenen Dosiermechanik und einem Ampulleneinschubteil, wie zum Beispiel einer Ampullenhülse oder auch einem Ampullenkörper, welcher beim Einschieben mit dem Sperrelement oder dem Dosierelement bzw. der Dosiermechanik zusammenwirken kann, also beispielsweise in Anlage mit dem Sperrelement oder der Dosiermechanik kommt, und bei einer durch die Einschubbewegung der Ampulle bedingten Bewegung oder Verschiebung der Dosiermechanik oder des Sperrelements relativ zur Injektionsvorrichtung oder zu einem Gehäuse der Injektionsvorrichtung ein Freigeben der Dosiermechanik oder des Dosierelementes bewirkt. Dabei kann das Sperrelement auch Teil der Dosiermechanik sein.

Vorzugsweise weist die Injektionsvorrichtung ein Führungselement, wie zum Beispiel eine Rampe oder ein relativ zur axialen Richtung schräg verlaufendes Profil auf, welches so relativ zu einem Halteelement des Sperrelements oder der Dosiermechanik angeordnet ist, dass eine axiale Verschiebung des Sperrelements oder der Dosiermechanik relativ zur Injektionsvorrichtung dazu führt, dass mindestens ein Halteelement durch die Führung so geführt wird, dass der Eingriff zwischen dem Sperrelement und dem Dosierelement oder der Dosiermechanik gelöst wird.

Vorzugsweise ist an der Injektionsvorrichtung ein Flansch vorgesehen, welcher beim Einbringen oder Eindrehen einer Zweikammerampulle gegen einen Stopfen der Ampulle drückt, so dass der Stopfen in die Ampulle beim Eindrehen der Ampulle in die Injektionsvorrichtung hineingedrückt wird und so ein Abmischen der in der Zweikammerampulle enthaltenen Substanzen erfolgt.

Gemäß einem weiteren Aspekt bezieht sich die Erfindung auf ein Verfahren zum Vorbereiten einer Injektionsvorrichtung zur Abgabe einer Substanz aus einer Zweikammerampulle, wobei die Zweikammerampulle in die Injektionsvorrichtung eingebracht und bevorzugt eingeschraubt wird und eine Sperrung oder Verdrehsicherung eines Einstellelements oder Aufzugelements der Injektionsvorrichtung dann gelöst wird, wenn die Ampulle so weit in die Injektionsvorrichtung eingebracht wird, dass die in der Zweikammerampulle enthaltenen Substanzen bevorzugt vollständig abgemischt werden.

Die Erfindung wird nachfolgend anhand eines bevorzugten Ausführungsbeispieles beschrieben werden. Es zeigen:
- Figur 1: eine Dosiermechanik mit einem gelösten Sperrelement in perspektivischer Ansicht;
- Figur 2: die in Figur 1 gezeigte Dosiermechanik vor dem Abmischen in Seitenansicht;
- Figur 2A: die in Figur 2 gezeigte Dosiermechanik entlang des Schnittes C-C;
- Figur 2B: das in Figur 2A gezeigte Detail D;
- Figur 3: die in Figur 2 gezeigte Dosiermechanik nach dem vollständigen Einschrauben und Abmischen der Zweikammerampulle;
- Figur 3A: die in Figur 3 gezeigte Dosiermechanik entlang des Schnittes A-A;
- Figur 3B: das in Figur 3A gezeigte Detail B;
- Figur 4: eine Draufsicht auf eine erfindungsgemäße Injektionsvorrichtung mit gesperrter Mechanik;
- Figur 4A: eine Schnittbildansicht entlang des Schnittes A-A in Figur 4;
- Figur 5: die in Figur 4 gezeigte Injektionsvorrichtung mit freigegebener Mechanik; und
- Figur 5A: eine Schnittbildansicht entlang des Schnittes B-B in Figur 5

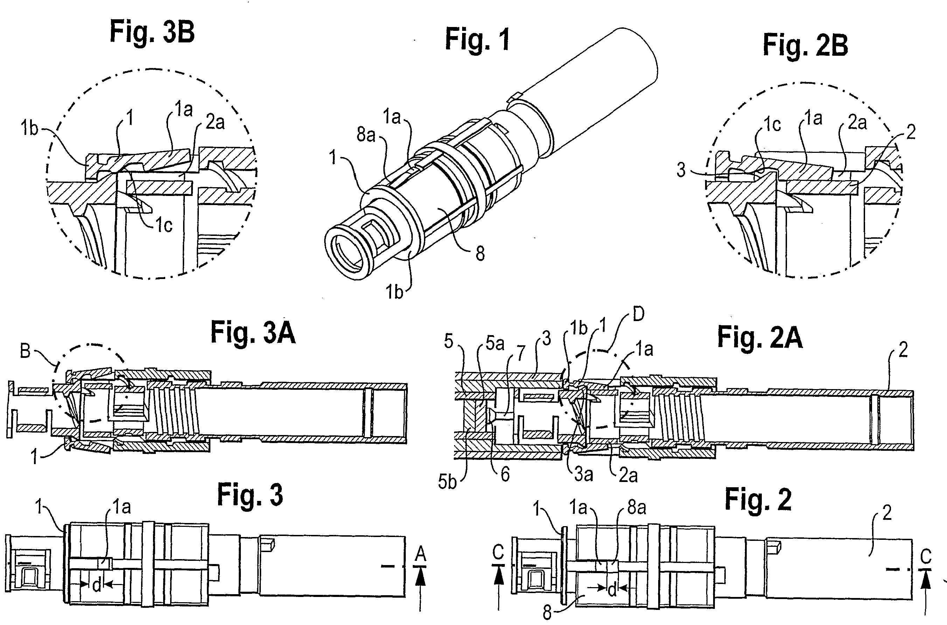

Figur 1 zeigt in perspektivischer Ansicht eine Ausführungsform einer erfindungsgemäßen Dosiermechanik, welche in eine Injektionsvorrichtung eingesetzt werden kann. Eine Sperrhülse, welche sich im eingesetzten Zustand der Dosiermechanik zumindest im vorderen oder distalen Teil innerhalb des Gehäuses der Injektionsvorrichtung befindet, oder auch das Gehäuse selbst, weist eine Durchbrechung 8a auf, in welche ein radial nach innen vorgespanntes Halteelement 1a des als Sperrelement vorgesehenen Sperrringes 1 nach dem Einschrauben der Ampulle 5 eingreift.

Der Sperrring 1 ist in der in Figur 2 gezeigten Ausgangsstellung zum Beispiel mittels eines Durchbruches im Gehäuse, in welchen ein Element des Sperrrings 1, wie zum Beispiel ein Halteelement 1a, eingreift, verdrehgesichert relativ zur Injektionsvorrichtung oder dem Gehäuse der Injektionsvorrichtung gelagert, so dass durch den Eingriff des Halteelementes 1a in die Durchbrechung oder Nut 2a der Dosierhülse 2 die Dosierhülse verdrehsichert in der Injektionsvorrichtung gelagert ist. Beispielsweise ist der Sperrring 1 in distale Richtung der Injektionsvorrichtung durch eine Federkraft beaufschlagt.

Figur 2 zeigt in Seitenansicht die in Figur 1 gezeigte Dosiermechanik, wobei das Halteelement 1a des Sperrringes 1 so relativ zur Nut 8a der Hülse 8 liegt, dass zwischen dem in Figur 2 rechts gezeigten proximalen Ende der Nut 8a noch der Abstand d besteht.

In Figur 2A ist die Schnittansicht entlang der Linie C-C in Figur 2 gezeigt, wobei zu sehen ist, dass das radial nach innen vorgespannte Halteelement 1a in eine Nut 2a der Dosierhülse 2 eingreift und so eine Drehbewegung der Dosierhülse 2 relativ zu dem Gehäuse 3 der Injektionsvorrichtung sperrt. Wird eine in einer Ampullenhülse 4 eingesetzte Ampulle 5 in die Injektionsvorrichtung eingeschraubt, um durch den aus der Injektionsvorrichtung an der Gewindestange 7 angebrachten Flansch 6 den hinteren oder proximalen Stopfen 5a der Ampulle in die Zweikammerampulle 5 einzuschieben, um so die Zweikammerampulle 5 abzumischen, so gelangt das in Figur 2A rechts gezeigte proximale Ende der Ampullenhülse 4 in Anlage an die vordere Fläche 1b des Sperrringes 1, wenn die Ampullenhülse 4 mit der darin liegenden Ampulle 5 so weit in die Injektionsvorrichtung eingeschraubt ist, dass der Flansch 6 soweit in die Ampulle 5 eingeschoben wurde, dass die Zweikammerampulle vollständig oder fast vollständig abgemischt ist. Wird die Ampullenhülse 4 weiter in die Injektionsvorrichtung eingedreht, so wird bedingt durch die Anlage des proximalen Endes der Ampullenhülse 4 an der Anlagefläche 1b der Sperrring 1 in Figur 2A nach rechts, also in proximale Richtung, verschoben. Dies bewirkt, dass das an dem Sperrring 1 vorgesehene Führungsprofil 1c in Anlage an die relativ zur Injektionsvorrichtung nicht verschiebbare Rampe 3a gelangt, und durch das Verschieben des Sperrringes 1 das Halteelement 1a entgegen der radial nach innen gerichteten Vorspannung des Halteelementes 1a nach außen gedrückt wird, wie in dem in Figur 3B gezeigten Detail B ersichtlich, wodurch der Eingriff des Halteelementes 1a in die Nut 2a der Dosierhülse 2 gelöst wird und die Dosierhülse 2 relativ zur Injektionsvorrichtung nicht mehr verdrehgesichert ist.

Die Figuren 3 und 3A zeigen den Zustand der Dosiermechanik nach dem Verschieben des Sperrringes 1 etwa um die Distanz d in proximale Richtung, um die Dosierhülse 2 zu entriegeln.

Nachdem die Ampulle 5 vollständig abgemischt wurde und infolgedessen die Verdrehsicherung 1a, 2a der Dosierhülse 2 gelöst wurde, kann die Dosierhülse 2 von einem Benutzer gedreht werden, um zum Beispiel eine Dosis einzustellen oder die Injektionsvorrichtung aufzuziehen, um eine Dosis bei einem Injektionsvorgang aus der Ampulle 5 abzugeben.

Die Mechanik-Sperrung der Einstellmechanik erfolgt somit durch die beiden gabelförmigen Sperrklinken 1a des Sperrrings, welche in entsprechende Vertiefungen 2a der Dreh- oder Dosierhülse 2 hineinragen. Da der Pen durch ein Drehen des Drehrings 2 geladen wird, ist dies nun nicht möglich, da die Verdrehung durch den Sperrring 1 verhindert wird.

Zum Entsperren der Mechanik wird die Ampullenhülse 4, welche zum Abmischen der Zweikammerampulle 5 in die Dosier- oder Einstellmechanik eingeschraubt wird, eingedreht. Auf den letzten ca. 2 mm des Einschraubvorganges wird der Sperrring 1 durch die Ampullenhülse 4 von der Sperrposition in die entsperrte Position gebracht. Hierfür besitzt der Sperrring 1 auf den Innenseiten der beiden gabelförmigen Sperrklinken 1a schräge Flächen, welche mit den schrägen Flächen 3a der Führungshülse oder des Gehäuses korrespondieren. Dadurch werden die beiden Sperrklinken 1a hinausgedrückt und geben somit die Dosierhülse 2 bzw. die Mechanik frei.

Das Halteelement 1a bzw. der Sperrring 1 ist dabei so ausgebildet, dass dieser durch die Ampullenhülse 4, welche beim Einschrauben der Ampulle 5 zum Abmischen der Substanz in den Pen eingeschraubt wird, in proximale Richtung verschoben wird. Eine zum Beispiel an dem Gehäuse des Pens vorgesehene Rampe oder Gleitfläche 3a bewirkt, dass das Halteelement 1a des Sperrrings 1, welches durch die Ampullenhülse 4 relativ zur Rampe 3a verschoben wird, radial nach außen gedrückt wird und somit die Verdrehsicherung der Dosierhülse 2 freigibt. Somit kann nach vollständigem Einschieben der Ampullenhülse 4 eine Aufdosierung mittels einer Drehung der Dosierhülse 2 vorgenommen werden.

Hierdurch kann sichergestellt werden, dass die Dosierhülse 2 erst dann gedreht werden kann, wenn die Ampullenhülse 4 vollständig in den Pen eingeschraubt wurde, das heißt, soweit bis die Ampullenhülse 4 an dem Sperrring 1 anschlägt und diesen ein Stück weit in die Injektionsvorrichtung hinein verschiebt.

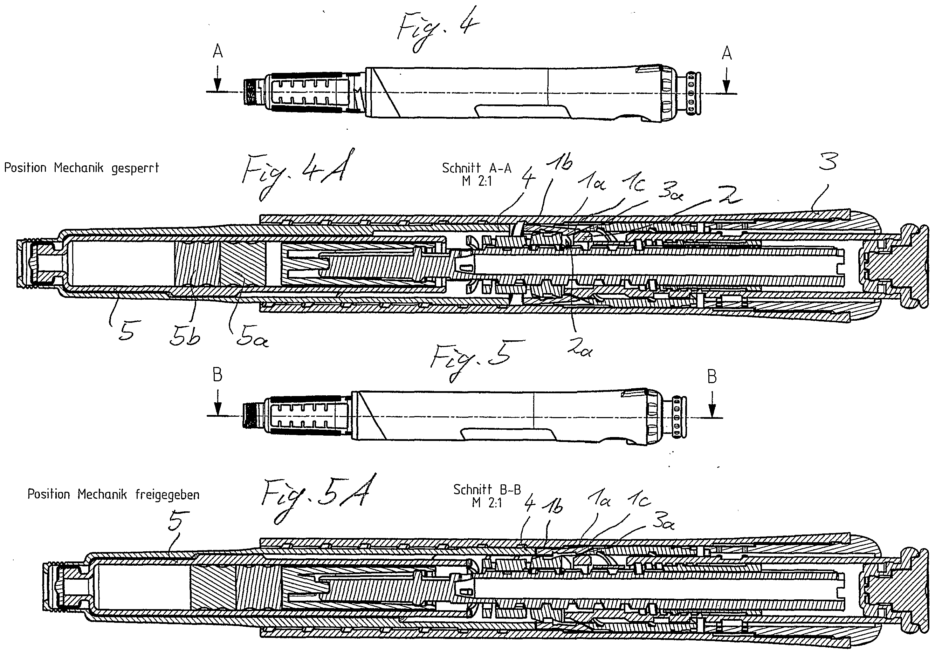

Figur 4 zeigt in Draufsicht eine Injektionsvorrichtung mit gesperrter Mechanik, welche entlang des Schnittes A-A in Figur 4A gezeigt ist. Wie aus Figur 4A ersichtlich, greift das radial nach innen vorgespannte Halteelement 1a, welches nicht zwangsläufig an einem Sperrring angebracht sein muss, in eine Nut 2a der Dosierhülse 2 ein und sperrt so eine Drehbewegung der Dosierhülse 2 relativ zu dem Gehäuse 3 der Injektionsvorrichtung. Die in die Ampullenhülse 4 eingesetzte Ampulle 5 kann in die Injektionsvorrichtung eingeschraubt werden, wie in den Figuren 5 und 5A gezeigt, wodurch, wenn das proximale Ende der Ampullenhülse 4 an die Anlagefläche 1b gelangt, das Führungsprofil 1c in Anlage an die relativ zur Injektionsvorrichtung nicht verschiebbare Rampe 3a gelangt und durch das Verschieben des Halteelementes 1a entgegen der radial nach innen gerichteten Vorspannung das Halteelement 1a nach außen gedrückt wird, wie aus Figur 5A ersichtlich. Hierdurch wird der Eingriff des Halteelementes 1a in die Nut 2a der Dosierhülse 2 gelöst und somit ist die Dosierhülse 2 relativ zur Injektionsvorrichtung nicht mehr verdrehgesichert. Bei der in Figur 5A gezeigten Injektionsvorrichtung ist die Ampulle somit vollständig oder fast vollständig eingebracht und eine Dosierung oder Einstellbewegung kann auf Grund der Freigabe der Dosierhülse 2 durch das Herausbewegen des Halteelementes 1a erfolgen.

## Patentansprüche

1. Dosiermechanik für eine Injektionsvorrichtung, mit
- einem Sperrelement (1) mit mindestens einem Halteelement (1a)
- einem Dosierelement (2), wobei
- das mindestens eine Halteelement (1a) mit dem Dosierelement (2) so zusammenwirken kann, dass eine Einstellbewegung des Dosierelements (2) in einer Ausgangsposition des Sperrelements (1) verhindert werden kann und erst nach einer Bewegung oder Verschiebung des Sperrelements (1) ermöglicht wird, **dadurch gekennzeichnet, dass** das mindestens eine Halteelement (1a) radial nach innen oder radial nach aussen vorgespannt ist, welches in eine Ausnehmung oder Nut (2a) des Dosierelements (2) eingreift.

2. Dosiermechanik nach Anspruch 1, wobei das Sperrelement (1) ringförmig ausgebildet ist und/oder eine Anschlagfläche (1b) für eine Ampulle (5) oder eine Ampullenhülse (4) aufweist.

3. Dosiermechanik nach einem der vorhergehenden Ansprüche mit mindestens zwei Halteelementen (1a).

4. Dosiermechanik nach einem der vorhergehenden Ansprüche, wobei das Dosierelement (2) durch das Sperrelement (1) lösbar arretiert oder festgestellt werden kann.

5. Dosiermechanik nach einem der vorhergehenden Ansprüche, wobei das Sperrelement (1) relativ zum Dosierelement (2) verschiebbar ist.

6. Injektionsvorrichtung mit einer Dosiermechanik nach einem der vorhergehenden Ansprüche und einer Ampulle (5) oder einem Ampulleneinschubelement oder einer Ampullenhülse (4), welche mit dem Sperrelement (1) so zusammenwirken können, dass das Sperrelement (1) nach einer vorgegebenen Einschubbewegung oder Einschubstrecke des Ampulleneinschubelement oder der Ampullenhülse (4) so bewegt oder verschoben wird, dass eine Kopplung (1a, 2a) zwischen dem Sperrelement (1) und dem Dosierelement (2) gelöst wird.

7. Injektionsvorrichtung nach dem vorhergehenden Anspruch mit einem Flansch (6), welcher mit einem Stopfen (5a) einer Zweikammerampulle (5) in Anlage kommen kann, um die Zweikammerampulle (5) beim Einbringen in die Injektionsvorrichtung abzumischen.

8. Injektionsvorrichtung nach einem der zwei vorhergehenden Ansprüche mit einem Führungsprofil oder einer Rampe (3a) an der Injektionsvorrichtung, an welchem ein Führungsprofil (1c) des Sperrelements (1) in Anlage kommen kann, um durch eine Relativbewegung des Sperrelements (1) zum Führungsprofil (3a) die Kopplung zwischen dem Sperrelement (1) und dem Dosierelement (2) zu lösen.

9. Injektionsvorrichtung nach einem der drei vorhergehenden Ansprüche, wobei das Sperrelement (1) verdrehgesichert in der Injektionsvorrichtung gelagert ist.

10. Injektionsvorrichtung nach einem der vier vorhergehenden Ansprüche, wobei das Sperrelement (1) entgegen einer Ampulleneinschubrichtung zum Beispiel durch ein Federelement vorgespannt ist.

11. Verfahren zum Vorbereiten einer Injektionsvorrichtung mit einer Dosiermechanik gemäss einem der Ansprüche 1 bis 5 oder einer Injektionsvorrichtung gemäss einem der Ansprüche 6 bis 10 zur Abgabe einer Substanz aus einer Ampulle oder Zweikammerampulle (5), wobei die Ampulle oder Zweikammerampulle (5) in die Injektionsvorrichtung eingebracht und bevorzugt eingeschraubt wird und eine Sperrung oder Verdrehsicherung eines Dosier- oder Einstellelementes (2) oder Aufzugelementes der Injektionsvorrichtung dann gelöst wird, wenn die Ampulle (5) so weit in die Injektionsvorrichtung eingebracht wurde, dass eine Substanz aus der Ampulle (5) definiert oder dosiert abgegeben werden kann oder dass die in der Zweikammerampulle (5) enthaltenen Substanzen bevorzugt vollständig abgemischt wurden.

## Claims

1. A dosing mechanism for an injection device comprising
- a locking element (1) having at least one holding element (1a),
- a dosing element (2), wherein
- the at least one holding element (1a) can cooperate with the dosing element (2) in such a way that a setting movement of the dosing element (2) can be prevented in a starting position of the locking element (1) and is permitted only after a movement or displacement of the locking element (1), **characterised in that** the at least one holding element (1a) is biased radially inwardly or radially outwardly, which engages into a recess or groove (2a) of the dosing element (2).

2. A dosing mechanism according to claim 1 wherein the locking element (1) is of an annular configuration and/or has an abutment surface (1b) for an ampoule (5) or an ampoule sleeve (4).

3. A dosing mechanism according to one of the preceding claims having at least two holding elements (1a).

4. A dosing mechanism according to one of the preceding claims wherein the dosing element (2) can be releasably arrested or fixed by the locking element (1).

5. A dosing mechanism according to one of the preceding claims wherein the locking element (1) is displaceable relative to the dosing element (2).

6. An injection device having a dosing mechanism according to one of the preceding claims and an ampoule (5) or an ampoule insertion element or an ampoule sleeve (4) which can cooperate with the locking element (1) in such a way that after a predetermined insertion movement or insertion distance of the ampoule insertion element or the ampoule sleeve (4) the locking element (1) can be so moved or displaced that a coupling (1a, 2a) between the locking element (1) and the dosing element (2) is released.

7. An injection device according to the preceding claim and comprising a flange (6) which can come into contact with a stopper (5a) of a two-chamber ampoule (5) in order to mix the two-chamber ampoule (5) upon introduction into the injection device.

8. An injection device according to one of the two preceding claims and having a guide profile member or a ramp (3a) at the injection device, at which a guide profile member (1c) of the locking element (1) can come into contact in order to release the coupling between the locking element (1) and the dosing element (2) by a relative movement of the locking elements (1) with respect to the guide profile member (3a).

9. An injection device according to one of the three preceding claims wherein the locking element (1) is mounted in the injection device in non-rotatable relationship.

10. An injection device according to one of the four preceding claims wherein the locking element (1) is biased for example by a spring element in opposition to an ampoule insertion direction.

11. A method of preparing an injection device having a dosing mechanism according to one of claims 1 to 5 or an injection device according to one of claims 6 to 10 for delivery of a substance from an ampoule or two-chamber ampoule (5), wherein the ampoule or two-chamber ampoule (5) is introduced into the injection device and preferably screwed therein and locking or non-rotational securing of a dosing or setting element (2) or uptake element of the injection device is released when the ampoule (5) has been so far introduced into the injection device that a substance can be discharged from the ampoule (5) in defined or dosed fashion or that the substances contained in the two-chamber ampoule (5) are preferably completely mixed.

## Revendications

1. Mécanisme de dosage pour un dispositif d'injection, avec
- un élément de blocage (1) avec au moins un élément de maintien (la),
- un élément de dosage (2), dans lequel
- l'au moins un élément de maintien (1a) peut coopérer avec l'élément de dosage (2) de telle sorte qu'un déplacement de réglage de l'élément de dosage (2) peut être empêché dans une position de départ de l'élément de blocage (1) et est rendu possible seulement après un déplacement ou coulissement de l'élément de blocage (1), **caractérisé en ce que** l'au moins un élément de maintien (1a) est précontraint radialement vers l'intérieur ou radialement vers l'extérieur, lequel vient en prise avec un évidement ou une rainure (2a) de l'élément de dosage (2).

2. Mécanisme de dosage selon la revendication 1, dans lequel l'élément de blocage (1) est réalisé de manière à présenter une forme annulaire et/ou présente une surface de butée (1b) pour une ampoule (5) ou une douille d'ampoule (4).

3. Mécanisme de dosage selon l'une quelconque des revendications précédentes avec au moins deux éléments de maintien (1a).

4. Mécanisme de dosage selon l'une quelconque des revendications précédentes, dans lequel l'élément de dosage (2) peut être arrêté ou immobilisé de manière amovible par l'élément de blocage (1).

5. Mécanisme de dosage selon l'une quelconque des revendications précédentes, dans lequel l'élément de blocage (1) peut être coulissé par rapport à l'élément de dosage (2).

6. Dispositif d'injection avec un mécanisme de dosage selon l'une quelconque des revendications précédentes, et une ampoule (5) ou un élément d'enfilage d'ampoule ou une douille d'ampoule (4), lesquels peuvent coopérer avec l'élément de blocage (1) de telle sorte que l'élément de blocage (1) est déplacé ou coulissé après un déplacement d'enfilage ou un trajet d'enfilage spécifié de l'élément d'enfilage d'ampoule ou de la douille d'ampoule (4) de telle sorte qu'un couplage (1a, 2a) entre l'élément de blocage (1) et l'élément de dosage (2) est desserré.

7. Dispositif d'injection selon la revendication précédente, avec une bride (6), laquelle peut venir en appui avec un bouchon (5a) d'une ampoule à deux chambres (5) pour mélanger l'ampoule à deux chambres (5) lors de l'introduction dans le dispositif d'injection.

8. Dispositif d'injection selon l'une quelconque des deux revendications précédentes avec un profil de guidage ou une rampe (3a) au niveau du dispositif d'injection, au niveau duquel un profil de guidage (1c) de l'élément de blocage (1) peut venir en appui pour desserrer par un déplacement relatif de l'élément de blocage (1) vers le profil de guidage (3a) le couplage entre l'élément de blocage (1) et l'élément de dosage (2).

9. Dispositif d'injection selon l'une quelconque des trois revendications précédentes, dans lequel l'élément de blocage (1) est monté de manière à empêcher toute rotation dans le dispositif d'injection.

10. Dispositif d'injection selon l'une quelconque des quatre revendications précédentes, dans lequel l'élément de blocage (1) est précontraint par exemple par un élément de ressort à l'encontre d'une direction d'enfilage d'ampoule.

11. Procédé servant à préparer un dispositif d'injection avec un mécanisme de dosage selon l'une quelconque des revendications 1 à 5 ou un dispositif d'injection selon l'une quelconque des revendications 6 à 10 pour distribuer une substance provenant d'une ampoule ou d'une ampoule à deux chambres (5), dans lequel l'ampoule ou l'ampoule à deux chambres (5) est introduite et de manière préférée est vissée dans le dispositif d'injection et un blocage ou un système de protection anti-rotation d'un élément de dosage ou de réglage (2) ou d'un élément de remontage du dispositif d'injection est desserré quand l'ampoule (5) a été introduite si loin dans le dispositif d'injection qu'une substance provenant de l'ampoule (5) peut être distribuée de manière définie ou dosée ou que les substances contenues dans l'ampoule à deux chambres (5) ont été mélangées de manière préférée en totalité.
